(19) Europäisches Patentamt
European Patent Office
Office européen des brevets

(11) Veröffentlichungsnummer: **0 065 723**
**B1**

(12) # EUROPÄISCHE PATENTSCHRIFT

(45) Veröffentlichungstag der Patentschrift: **12.09.84**

(21) Anmeldenummer: **82104259.5**

(22) Anmeldetag: **15.05.82**

(51) Int. Cl.³: **C 07 D 285/06**, C 07 D 277/56, C 07 D 285/08, C 07 D 285/10, C 07 D 285/12, C 07 D 275/02, A 01 N 43/82, A 01 N 43/78, A 01 N 43/80

(54) **N-substituierte 2-Methylnaphthylamide, Verfahren zu ihrer Herstellung und ihre Verwendung zur Bekämpfung von Pilzen.**

(30) Priorität: **25.05.81 DE 3120804**

(43) Veröffentlichungstag der Anmeldung: **01.12.82 Patentblatt 82/48**

(45) Bekanntmachung des Hinweises auf die Patenterteilung: **12.09.84 Patentblatt 84/37**

(84) Benannte Vertragsstaaten: **AT BE CH DE FR GB IT LI NL SE**

(56) Entgegenhaltungen:
**EP - A - 0 019 742**
**EP - A - 0 046 497**
**EP - A - 0 051 742**
**DE - A - 2 804 299**
**FR - A - 2 143 364**
**FR - A - 2 451 371**

(73) Patentinhaber: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Zeeh, Bernd, Dr., Thorwaldsenstrasse 5, D-6700 Ludwigshafen (DE)**
Erfinder: **Ammermann, Eberhard, Dr., Sachsenstrasse 3, D-6700 Ludwigshafen (DE)**
Erfinder: **Hamprecht, Gerhard, Dr., Rote-Turm-Strasse 28, D-6940 Weinheim (DE)**
Erfinder: **Pommer, Ernst-Heinrich, Dr., Berliner Platz 7, D-6703 Limburgerhof (DE)**

**EP 0 065 723 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft N-substituierte 2-Methylnaphthylamide, Verfahren zu ihrer Herstellung und Fungizide, die diese Verbindungen als Wirkstoffe enthalten.

Es ist bereits bekannt, das Zink-ethylen-1,2-bisdithiocarbamat, das N-Trichlormethylthiophthalimid und N-Trichlormethylthio-tetrahydrophthalimid als Fungizide in der Landwirtschaft und im Gartenbau verwendet werden können. Die genannten Verbindungen sind gute Mittel zur Bekämpfung von pilzlichen Krankheiten [R. Wegler, Chemie der Pflanzenschutz- und Schädlingsbekämpfungsmittel, Band 2, S. 65 bis 66 und 109, und Band 4, S. 139 und 191, Springer-Verlag, Berlin/Heidelberg/New York, (1970) und (1977)]. Jedoch sind diese Fungizide nicht nach erfolgter Infektion verwendbar, und ihre Wirkung bei niedrigen Aufwandkonzentrationen genügt nicht den Anforderungen der Praxis.

Es ist ferner bekannt, Säureanilide, die den Tetrahydrofuranonrest enthalten, als Fungizide zu verwenden (DE-A Nr. 2804299). Hierdurch werden die neuen Verbindungen gemäss der Erfindung, die weder den Anilinrest noch den Tetrahydrofuranonrest enthalten, nicht nahegelegt.

Es ist ferner bekannt, Säureanilide, die den 1,2,3-Thiadiazol-5-carbonsäurerest enthalten, als Fungizide zu verwenden (FR-A Nr. 2451371). Hierdurch werden die neuen Verbindungen gemäss der Erfindung, die keinen 1,2,3-Thiadiazol-5-carbonsäurerest enthalten, nicht nahegelegt.

Es wurde gefunden, dass N-substituierte 2-Methyl-naphthylamide der Formel

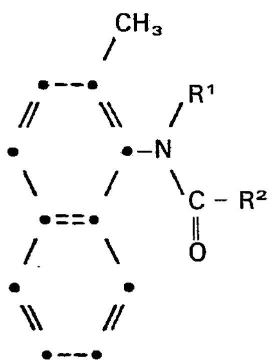 (I)

in der

$R^1$ für den Rest $-CH(CH_3)-COOCH_3$ steht, und

$R^2$ für einen 5gliedrigen, ein Schwefelatom und bis zu zwei Stickstoffatome enthaltenden, gegebenenfalls durch Methyl oder Ethyl substituierten heterocyclischen Ring, ausser dem 1,2,3-Thiadiazol-5-yl-rest steht, starke fungizide Eigenschaften aufweisen.

In der Formel I steht $R^2$ bevorzugt für 3-, 4- oder 5-Isothiazolyl, 3-Methyl-4-isothiazolyl, 3-Methyl-5-isothiazolyl, 2-, 4- oder 5-Thiazolyl, 4-Methyl-5-thiazolyl, 1,2,3-Thiadiazol-4-yl, 1,2,4-Thiadiazol-3-yl, 1,2,4-Thiadiazol-5-yl, 1,2,5-Thiadiazol-3-yl, 1,3,4-Thiadiazol-2-yl.

Die N-substituierten 2-Methylnaphthylamide der Formel I besitzen ein chirales Kohlenstoffatom in dem Rest $R^1$. Die optisch reinen Enantiomeren bzw. die Diastereomeren können nach üblichen Methoden erhalten werden. Die Formel I erfasst auch diese Verbindungen in reiner Form oder in Form von Mischungen. Als Fungizide sind sowohl die reinen Enantiomeren bzw. die einheitlichen Diastereomeren als auch die bei der Synthese üblicherweise anfallenden Mischungen wirksam.

Man erhält die Verbindungen der Formel I durch Umsetzung von 2-Methylnaphthylaminen der Formel

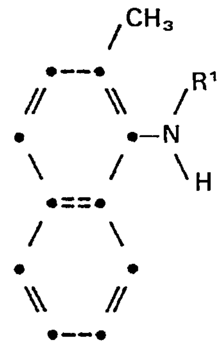 (II)

in der

$R^1$ die obengenannten Bedeutungen hat,

a) mit einem Säurehalogenid der Formel

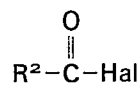 (III)

in der

$R^2$ die obengenannten Bedeutungen hat und Hal Chlor oder Brom bedeutet, oder

b) mit einem Säureanhydrid der Formel

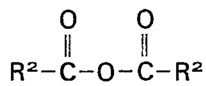 (IV)

in der

$R^2$ die obengenannten Bedeutungen hat, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen −10 und +100° C.

Als gegenüber den Reaktionsteilnehmern inerte Lösungs- oder Verdünnungsmittel können beispielsweise aliphatische oder aromatische Kohlenwasserstoffe, wie Pentan, Cyclohexan, Petrolether, Benzol, Toluol oder Xylole; Halogenkohlenwasserstoffe, wie Methylenchlorid, Chloroform, 1,2-Dichlorethan, Chlorbenzol; Ketone, wie Aceton und Methylethylketon; Ether, wie Diethylether, Dimethoxyethan, Tetrahydrofuran oder Dioxan; Ester, wie Essigsäureethylester; Nitrile, wie Acetonitril; Sulfoxide, wie Dimethylsulfoxid; oder entsprechende Gemische verwendet werden.

Geeignete anorganische oder organische Basen, die als säurebindende Mittel bei der Reaktion verwendet werden können, sind beispielsweise Alkalimetall- und Erdalkalimetallcarbonate, wie Natrium- oder Kaliumbicarbonat, Natrium- oder Kaliumcarbonat, Calciumcarbonat; Borate, wie Natriumborat; Phosphate, wie Natrium- oder Kaliumdi- oder -triphosphat; oder Amine, wie

Triethylamin, N,N-Dimethylanilin, N,N-Dimethylcyclohexylamin, N-Methylpiperidin oder Pyridin. Es können aber auch andere übliche Basen verwendet werden. Die Menge an Base pro Mol 2-Methylnaphthylamin der Formel II beträgt zweckmässigerweise 1 bis 10 mol.

Die Umsetzung mit dem Säurehalogenid der Formel III kann auch ohne säurebindende Mittel durchgeführt werden, wobei in einigen Fällen das Durchleiten von trockenem Stickstoff zur Vertreibung des gebildeten Halogenwasserstoffs angezeigt ist.

Als Reaktionsbeschleuniger kommen vorzugsweise Metallhalogenide, wie Natriumbromid oder Kaliumjodid, Azole, wie Imidazol oder 1,2,4-Triazol, Pyridine, wie 4-Dimethylaminopyridin, oder Dimethylformamid, in Betracht. Der Reaktionsbeschleuniger wird zweckmässigerweise in Mengen von 0,1 bis 1 mol, bezogen auf 1 mol 2-Methylnaphthylamin der Formel II zugesetzt.

Die erfindungsgemässen Umsetzungen erfolgen beispielsweise bei Temperaturen zwischen −10 und +100° C, vorzugsweise zwischen 0 und +40° C, drucklos oder unter Druck, kontinuierlich oder diskontinuierlich.

Die Ausgangsstoffe der allgemeinen Formel II sind zum Teil bekannt (DE-OS Nr. 2845454, EP-OS Nr. 18510) und können nach an sich allgemein bekannten Methoden hergestellt werden.

Die Herstellung der neuen Verbindungen wird durch folgende Beispiele erläutert:

*Beispiel 1:*

Herstellung von

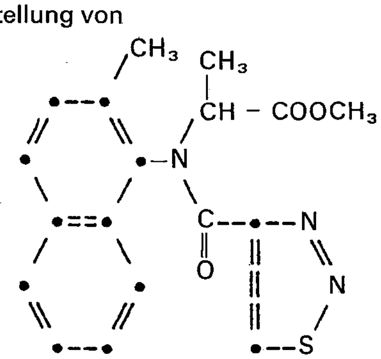

Zu einer Lösung von 22,9 g (0,0945 mol) N-(2-Methyl-1-naphthyl)-alaninmethylester in 100 ml trockenem Toluol werden 14 g (0,095 mol) 1,2,3-Thiadiazol-4-carbonylchlorid in 30 ml Toluol zwischen +15 und +30° C zugetropft. Das Gemisch wird bei 80° C 8 h lang nachgerührt. Dabei wird der entstehende Chlorwasserstoff in einem schwachen Stickstoffatom ständig aus dem Reaktionsgemisch vertrieben. Nach dem Abkühlen wird das Gemisch 1 h lang mit der Lösung von 5 g Natriumhydrogencarbonat in 100 ml Wasser gerührt, die organische Phase wird getrennt, über Natriumsulfat getrocknet, mit Kohle entfärbt und unter vermindertem Druck eingedampft. Der verbleibende ölige Rückstand wird 3 h lang bei 50° C und 0,1 mbar getrocknet. Man erhält 32 g (95,2% d.Th.) N-(1,2,3-Thiadiazol-4-yl-carbonyl)-N-(2-methyl-1-naphthyl)-alaninmethylester als hellbraunes Harz.

IR-Spektrum (KBr): 3110, 3060, 3000, 2990, 2955, 1726, 1645, 1496, 1404, 1320, 1282, 1250, 1240, 1128, 1105, 1075, 967, 870, 822, 788, 760 $cm^{-1}$.

Analog können beispielsweise folgende Verbindungen hergestellt werden:

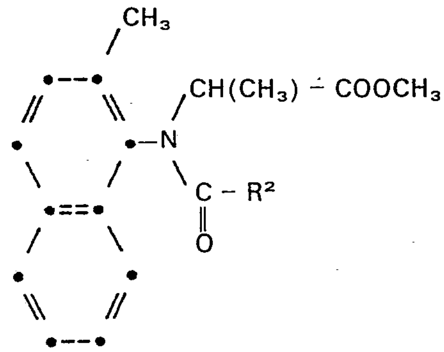

| Nr. | $R^2$ | Physikal. Daten |
|---|---|---|
| 4 | 1,2-Thiazol-4-yl | Öl |
| 6 | 1,2-Thiazol-5-yl | |
| 7 | 3-Methyl-1,2-thiazol-4-yl | Öl |
| 8 | 1,3-Thiazol-2-yl | Fp. 86-88° C |
| 10 | 1,3-Thiazol-4-yl | Fp. 118-120° C |
| 12 | 1,3-Thiazol-5-yl | |
| 13 | 5-Methyl-1,3-thiazol-4-yl | Fp. 112-115° C |
| 16 | 1,3,4-Thiadiazol-2-yl | Öl |
| 17 | 1,2,5-Thiadiazol-3-yl | Harz |

Die N-substituierten 2-Methylnaphthylamide der Formel I zeigen eine fungitoxische Wirksamkeit gegen phytopathogene Pilze, insbesondere aus der Klasse der Phycomyceten. Die Verbindungen sind daher beispielsweise geeignet zur Bekämpfung von *Phytophthora infestans* an Tomaten und Kartoffeln, *Phytophthora parasitica* an Erdbeeren, *Phytophtora cactorum* an Äpfeln, *Pseudoperonospora cubenis* an Gurken, *Pseudoperonospora humuli* an Hopfen, *Peronospora destructor* an Zwiebeln, *Peronospora sparsa* an Rosen, *Peronospora tabacina* an Tabak, *Plasmopara viticola* an Reben, *Plasmopara halstedii* an Sonnenblumen, *Sclerospora macrospora* an Mais, *Bremia lactucae* an Salat, *Mucor mucedo* an Früchten, *Rhizopus nigricans* an Rüben, *Erysiphe graminis* an Getreide, *Uncinula necator* an Reben, *Podosphaera leucotricha* an Äpfeln, *Sphaerotheca fuliginea* an Rosen, *Erysiphe cichoracearum* an Gurken.

Die Aufwandmengen liegen je nach Art des gewünschten Effektes zwischen 0,1 und 5 kg Wirkstoff je ha. Ein Teil der Wirkstoffe zeigt kurative Eigenschaften, d.h. die Anwendung der Mittel kann noch nach erfolgter Infektion der Pflanzen durch die Krankheitserreger vorgenommen werden, um einen sicheren Bekämpfungserfolg zu erzielen. Darüber hinaus sind viele der neuen Verbindungen systemisch wirksam, so dass über die Wurzelbehandlung auch ein Schutz oberirdischer Pflanzenteile möglich ist.

Die neuen Wirkstoffe werden beispielsweise in Form von direkt versprühbaren Lösungen, Pulvern, Suspensionen, auch hochprozentige wässerige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Giessen angewendet. Die Anwendungsformen richten sich ganz nach den Verwendungszwecken; sie sollten in jedem Fall möglichst die feinste Verteilung der neuen Wirkstoffe gewährleisten. Diese Formulierungen enthalten 0,1 bis 95 Gew.-%, vorzugsweise 0,5 bis 90 Gew.-%, Wirkstoff.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle usw. sowie Öle planzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser usw. in Betracht.

Wässerige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol-, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nussschalenmehle, Cellulosepulver und andere feste Trägerstoffe.

*Beispiele für Zubereitungen sind:*

I. Man vermischt 90 Gewichtsteile der Verbindung 1 mit 10 Gewichtsteilen N-Methyl-alphapyrrolidon und erhält eine Lösung, die zur Anwendung in Form kleinster Tropfen geeignet ist.

II. 20 Gewichtsteile der Verbindung 10 werden in einer Mischung gelöst, die aus 80 Gewichtsteilen Xylol, 10 Gewichtsteilen des Anlagerungsproduktes von 8 bis 10 mol Ethylenoxid an 1 mol Ölsäure-N-monoethanolamid, 5-Gewichtsteilen Calciumsalz der Dodecylbenzolsulfonsäure und 5 Gewichtsteilen des Anlagerungsproduktes von 40 mol Ethylenoxid an 1 mol Ricinusöl besteht. Durch Ausgiessen und feines Verteilen der Lösung in 100 000 Gewichtsteilen Wasser erhält man eine wässerige Dispersion, die 0,02 Gew.-% des Wirkstoffs enthält.

III. 20 Gewichtsteile der Verbindung 13 werden in einer Mischung gelöst, die aus 40 Gewichtsteilen Cyclohexanon, 30 Gewichtsteilen Isobutanol, 20 Gewichtsteilen des Anlagerungsproduktes von 40 mol Ethylenoxid an 1 mol Ricinusöl besteht. Durch Eingiessen und feines Verteilen der Lösung in 100 000 Gewichtsteilen Wasser erhält man eine wässerige Dispersion, die 0,02 Gew.-% des Wirkstoffs enthält.

IV. 20 Gewichtsteile der Verbindung 17 werden in einer Mischung gelöst, die aus 25 Gewichtsteilen Cyclohexanol, 65 Gewichtsteilen einer Mineralölfraktion vom Siedepunkt 210 bis 280° C und 10 Gewichtsteilen des Anlagerungsproduktes von 40 mol Ethylenoxid an 1 mol Ricinusöl besteht. Durch Eingiessen und feines Verteilen der Lösung in 100 000 Gewichtsteilen Wasser erhält man eine wässerige Dispersion, die 0,02 Gew.-% das Wirkstoffs enthält.

V. 20 Gewichtsteile der Verbindung 13 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

VI. 3 Gewichtsteile der Verbindung 8 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung 10 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung 13 werden mit 10 Teilen Natriumsalz eines Phenolsulfonsäure-harnstoffformaldehyd-Kondensates, 2 Teilen Kieselgel und 48 Teilen Wasser innig vermischt. Man erhält eine stabile wässerige Dispersion. Durch Verdünnen mit 100 000 Gewichtsteilen Wasser erhält man eine wässerige Dispersion, die 0,04 Gew.-% Wirkstoff enthält.

IX. 20 Teile der Verbindung 13 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemässen Wirkstoffe können auch zusammen mit anderen Wirkstoffen, z.B. Herbiziden, Insektiziden, Wachstumsregulatoren und anderen Fungiziden oder auch mit Düngemitteln vermischt und ausgebracht werden. In vielen Fällen erhält man bei der Mischung mit Fungiziden auch eine Vergrösserung des fungiziden Wirkungsspektrums; bei einer Anzahl dieser Fungizidmischungen treten auch synergistische Effekte auf, d.h. die fungizide Wirksamkeit des Kombinationsproduktes ist grösser als die der addierten Wirksamkeiten der Einzelkomponenten. Eine besonders günstige Vergrösserung des Wirkungsspektrums wird mit folgenden fungiziden Wirkstoffen erzielt:

Manganethylenbisdithiocarbamat
Mangan-Zinkethylenbisdithiocarbamat
Ammoniak-komplex von Zink-(N,N-ethylen-bis-dithiocarbamat)
N-Trichlormethylthio-tetrahydrophthalimid
N-Trichlormethyl-phthalimid
5-Ethoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die folgende Liste von fungiziden Wirkstoffen, mit denen die erfindungsgemässen Verbindungen kombiniert werden können, soll die Kombinationsmöglichkeiten erläutern, nicht aber einschränken. Wirkstoffe, die mit den erfindungsgemässen Verbindungen kombiniert werden können, sind beispielsweise:

Dithiocarbamate und deren Derivate, wie
Ferridimethyldithiocarbamat,
Zinkdimethyldithiocarbamat,
Zinkethylenbisthiocarbamat,
Tetramethylthiuramdisulfide,
Zink-(N,N-propylen-bis-dithiocarbamat),
Ammoniak-Komplex von Zink-(N,N-propylen-bis-dithiocarbamat), und
N,N'-Polyethylen-bis-(thiocarbamoyl)-disulfid;
Nitrophenolderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenylisopropylcarbonat;
heterocyclische Strukturen, wie
2-Heptadecyl-2-imidazol-acetat,
2,4-Dichlor-6-(o-chloranilino)-s-triazin,
O,O-Diethyl-phthalimidophosphonthionat,
5-Amino-1-(bis-(dimethylamino)-phosphinyl)-3-phenyl-1,2,4-triazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2)-benzimidazol,
Piperazin-1,4-diyl-bis-1-(2,2,2-trichlor-ethyl)-formamid,
2-Thiazolyl-(4)-benzimidazol,
5-Butyl-2-dimethylamino-4-hydroxy-6-methylpyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol;
sowie verschiedene Fungizide, wie
Dodecylguanidinacetat,
3-[2-(3,5-Dimethyl-2-oxycyclohexyl)-2-hydroxyethyl]-glutarimid,
Hexachlorbenzol,
N-Dichlorfluormethylthio-N,N'-dimethyl-N-phenyl-schwefelsäurediamid,
2,5-Dimethyl-furan-3-carbonsäureanilid,
2-Methyl-benzoesäure-anilid,
2-Jod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,

1-(4-Chlorphenoxy)-3,3-dimethyl-1-(1H-1,2,4-triazol-1-yl)-2-butanon,
α-(2-Chlorphenyl)-α-(4-chlorphenyl)-5-pyrimidinmethanol.

Für die biologischen Versuche werden die folgenden Verbindungen als Vergleichssubstanzen verwendet:
N-Trichlormethylthio-phthalimid (A),
N-Trichlormethylthio-tetrahydrophthalimid (B),
Zink-ethylen-1,2-bis-dithiocarbamat (C).

*Versuch 1:*

Wirksamkeit gegen Auflaufkrankheiten an Erbsen

100 g Proben Erbsensaatgut der Sorte „Senator" werden in Glasflaschen etwa 5 min lang mit 300 mg (= 0,3 Gew.-%) Beizmittelaufbereitungen, die 40% Wirkstoff in der Trockensubstanz enthalten, sorgfältig gebeizt. Danach werden jeweils 100 Samen in Saatkisten 3 cm tief und mit einem Abstand von 3 bis 5 cm in eine Komposterde eingesät, die eine starke natürliche Verseuchung mit den Pilzen *Pythium spec.*, *Aphanomyces spec.* und *Fusarium oxysporum* aufweist. Die Kästen werden im Gewächshaus bei Temperaturen von 17 bis 20° C aufgestellt. Nach einer Versuchsdauer von 21 Tagen wird die Anzahl gesunder Erbsenpflanzen ermittelt.

In diesem Test zeigen die Wirkstoffe Nrn. 1, 10 und 17 eine bessere fungizide Wirkung als die Vergleichssubstanz B.

*Versuch 2:*

Wirksamkeit gegen Gurkenmehltau

Blätter von in Töpfen gewachsenen Gurkenkeimlingen der Sorte „Chinesische Schlange" werden im Zweiblattstadium mit einer Sporensuspension des Gurkenmehltaus *(Erysiphe cichoracearum)* besprüht. Nach dem Antrocknen über Nacht werden die Versuchspflanzen mit wässerigen Emulsionen aus 80% Wirkstoff und 20% Emulgiermittel besprüht. Nach dem Antrocknen des Spritzbelages werden sie anschliessend im Gewächshaus bei Temperaturen zwischen 20 und 22° C und 70-80% relativer Luftfeuchtigkeit aufgestellt. Zur Beurteilung der Wirksamkeit der neuen Wirkstoffe wird das Ausmass der Mehltaupilzentwicklung nach 8 Tagen ermittelt.

In diesem Test zeigen die Wirkstoffe Nrn. 1 und 17 eine sehr gute Wirkung.

*Versuch 3:*

Wirksamkeit gegen *Phytophthora infestans* an Tomaten

Blätter von Topfpflanzen der Sorte „Grosse Fleischtomate" werden mit wässerigen Suspensionen, die 0,025% (Gewichtsprozent) Wirkstoff enthalten, besprüht. Nach dem Antrocknen des Spritzbelages werden die Blätter mit einer Zoosporenaufschwemmung des Pilzes *Phytophthora infestans* infiziert. Die Pflanzen werden dann in einer wasserdampfgesättigten Kammer bei Temperaturen zwischen 16 und 18° C aufgestellt. Nach 5 Tagen hat sich die Krankheit auf den unbehandelten, jedoch infizierten Kontrollpflanzen so stark entwickelt, dass die fungizide Wirksamkeit der Substanzen beurteilt werden kann.

In diesem Test zeigen die Wirkstoffe Nrn. 1, 8, 10, 12 eine bessere Wirkung als die Vergleichssubstanz C.

*Versuch 4:*

Wirksamkeit gegen *Plasmopara viticola*

Blätter von Topfreben der Sorte „Müller-Thurgau" werden mit wässerigen Suspensionen, die 0,025% (Gewichtsprozent) Wirkstoff enthalten, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von *Plasmopara viticola* (Rebenperonospora) infiziert. Danach kommen die Reben zunächst für 16 h in eine wasserdampfgesättigte Kammer bei 24° C und anschliessend 8 Tage in ein Gewächshaus mit Temperaturen zwischen 20 und 30° C. Nach dieser Zeit werden die Pflanzen zur Beschleunigung des Sporangienträgerausbruches abermals während 16 h in der feuchten Kammer aufgestellt. Dann erfolgt die Beurteilung des Ausmasses des Pilzausbruches auf den Blattunterseiten.

In diesem Test zeigen die Wirkstoffe Nrn. 1, 10, 13, 17 und 4 eine bessere Wirkung als die Vergleichssubtanz A.

**Patentansprüche** für die Vertragsstaaten BE, CH, DE, FR, GB, IT, LI, NL, SE

1. N-substituiertes 2-Methylnaphthylamid der Formel

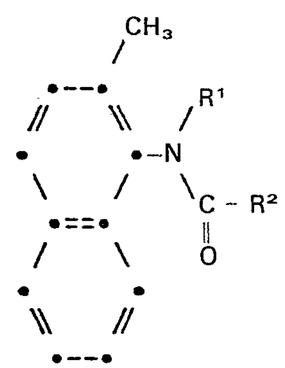

(I)

in der

$R^1$ für den Rest $-CH(CH_3)-COOCH_3$ steht,

$R^2$ für einen 5gliedrigen, ein Schwefelatom und bis zu zwei Stickstoffatome enthaltenden, gegebenenfalls durch Methyl oder Ethyl substituierten heterocyclischen Ring ausser dem 1,2,3-Thiadiazol-5-yl-rest steht.

2. Fungizid, enthaltend ein N-substituiertes 2-Methylnaphthylamid der Formel (I) gemäss Anspruch 1.

3. Fungizid, enthaltend einen festen oder flüssigen Trägerstoff und ein N-substituiertes 2-Methylnaphthylamid der Formel (I) gemäss Anspruch 1.

4. Verfahren zur Herstellung eines Fungizids, dadurch gekennzeichnet, dass man einen festen oder flüssigen Trägerstoff vermischt mit einem N-

substituierten 2-Methylnaphthylamid der Formel (I) gemäss Anspruch 1.

5. Verfahren zur Bekämpfung von Pilzen, dadurch gekennzeichnet, dass man die Pilze oder die vor Pilzbefall zu schützenden Gegenstände behandelt mit einem N-substituierten 2-Methylnaphthylamid der Formel (I) gemäss Anspruch 1.

6. Verfahren zur Herstellung eines N-substituierten 2-Methylnaphthylamids der Formel (I) gemäss Anspruch 1, dadurch gekennzeichnet, dass man ein 2-Methylnaphthylamid der Formel

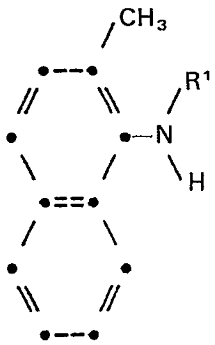

(II)

in der

$R^1$ die im Anspruch 1 angegebenen Bedeutungen hat,

a) mit einem Säurehalogenid der Formel

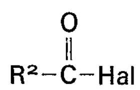

(III)

in der

$R^2$ die im Anspruch 1 angegebenen Bedeutungen hat und Hal Chlor oder Brom bedeutet, oder

b) mit einem Säureanhydrid der Formel

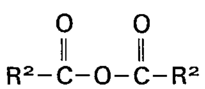

(IV)

in der

$R^2$ die im Anspruch 1 angegebenen Bedeutungen hat, gegebenenfalls in Gegenwart eines Lösungsmittels, gegebenenfalls unter Zusatz einer anorganischen oder organischen Base und gegebenenfalls unter Zusatz eines Reaktionsbeschleunigers, bei Temperaturen zwischen −10 und +100° C umsetzt.

7. N-substituierte 2-Methylnaphthylamide der Formel (I) gemäss Anspruch 1, dadurch gekennzeichnet, dass $R^2$ für 3-, 4- oder 5-Isothiazolyl, 3-Methyl-4-isothiazolyl, 3-Methyl-5-isothiazolyl, 2-, 4- oder 5-Thiazolyl, 4-Methyl-5-thiazolyl, 1,2,3-Thiadiazol-4-yl, 1,2,4-Thiadiazol-3-yl, 1,2,4-Thiadiazol-5-yl, 1,2,5-Thiadiazol-3-yl, 1,3,4-Thiadiazol-2-yl steht.

8. Fungizid, enthaltend ein N-substituiertes 2-Methylnaphthylamid der Formel (I) gemäss Anspruch 1, dadurch gekennzeichnet, dass $R^2$ für 3-, 4- oder 5-Isothiazolyl, 3-Methyl-4-isothiazolyl, 3-Methyl-5-isothiazolyl, 2-, 4- oder 5-Thiazolyl, 4-Methyl-5-thiazolyl, 1,2,3-Thiadiazol-4-yl, 1,2,4-Thiadiazol-3-yl, 1,2,4-Thiadiazol-5-yl, 1,2,5-Thiadiazol-3-yl, 1,3,4-Thiadiazol-2-yl steht.

**Patentansprüche** für den Vertragsstaat: AT

1. Fungizid, enthaltend ein N-substituiertes 2-Methylnaphthylamid der Formel

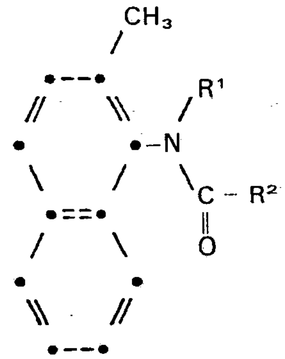

(I)

in der

$R^1$ für den Rest $-CH(CH_3)-COOCH_3$ steht,

$R^2$ für einen 5gliedrigen, ein Schwefelatom und bis zu zwei Stickstoffatome enthaltenden, gegebenenfalls durch Methyl oder Ethyl substituierten heterocyclischen Ring ausser dem 1,2,3-Thiadiazol-5-yl-rest steht.

2. Fungizid, enthaltend einen festen oder flüssigen Trägerstoff und ein N-substituiertes 2-Methylnaphthylamid der Formel (I) gemäss Anspruch 1.

3. Verfahren zur Herstellung eines Fungizids, dadurch gekennzeichnet, dass man einen festen oder flüssigen Trägerstoff vermischt mit einem N-substituierten 2-Methylnaphthylamid der Formel (I) gemäss Anspruch 1.

4. Verfahren zur Bekämpfung von Pilzen, dadurch gekennzeichnet, dass man die Pilze oder die vor Pilzbefall zu schützenden Gegenstände behandelt mit einem N-substituierten 2-Methylnaphthylamid der Formel (I) gemäss Anspruch 1.

5. Verfahren zur Herstellung eines N-substituierten 2-Methylnaphthylamids der Formel (I) gemäss Anspruch 1, dadurch gekennzeichnet, dass man ein 2-Methylnaphthylamid der Formel

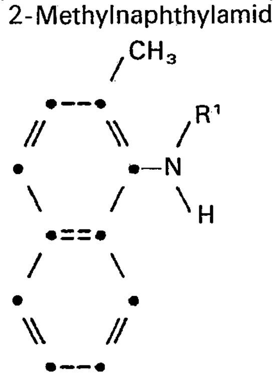

(II)

in der

$R^1$ die im Anspruch 1 angegebenen Bedeutungen hat,

a) mit einem Säurehalogenid der Formel

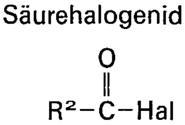

(III)

in der

$R^2$ die im Anspruch 1 angegebenen Bedeutungen hat und Hal Chlor oder Brom bedeutet, oder

b) mit einem Säureanhydrid der Formel

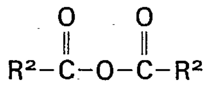 (IV)

in der

$R^2$ die im Anspruch 1 angegebenen Bedeutungen hat, gegebenenfalls in Gegenwart eines Lösungsmittels, gegebenenfalls unter Zusatz einer anorganischen oder organischen Base und gegebenenfalls unter Zusatz eines Reaktionsbeschleunigers, bei Temperaturen zwischen −10 und +100° C umsetzt.

6. Fungizid, enthaltend ein N-substituiertes 2-Methylnaphthylamid der Formel (I), dadurch gekennzeichnet, dass $R^2$ für 3-, 4- oder 5-Isothiazolyl, 3-Methyl-4-isothiazolyl, 3-Methyl-5-isothiazolyl, 2-, 4- oder 5-Thiazolyl, 4-Methyl-5-thiazolyl, 1,2,3-Thiadiazol-4-yl, 1,2,4-Thiadiazol-3-yl, 1,2,4-Thiadiazol-5-yl, 1,2,5-Thiadiazol-3-yl, 1,3,4-Thiadiazol-2-yl steht.

**Claims** for the Contracting States BE, CH, DE, FR, GB, IT, LI, NL, SE

1. An N-substituted 2-methylnaphthylamide of the Formula

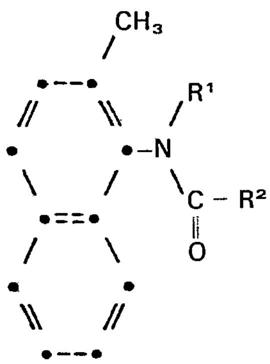 (I)

where $R^1$ is $-CH(CH_3)-COOCH_3$, and $R^2$ is a 5-membered heterocyclic ring which contains a sulfur atom and not more than two nitrogen atoms and is unsubstituted or substituted by methyl or ethyl, with the exception of 1,2,3-thiadiazol-5-yl.

2. A fungicide containing an N-substituted 2-methylnaphtylamide of the Formula (I) as claimed in Claim 1.

3. A fungicide containing a solid or liquid carrier and an N-substituted 2-methylnaphthylamide of the Formula (I) as claimed in Claim 1.

4. A process for producing a fungicide, wherein a solid or liquid carrier is mixed with an N-substituted 2-methylnaphthylamide of the Formula (I) as claimed in Claim 1.

5. Process for combating fungi, wherein the fungi or the objects to be protected against fungus attack are treated with an N-substituted 2-methylnaphthylamide of the Formula (I) as claimed in Claim 1.

6. A process for preparing an N-substituted 2-methylnaphthylamide of the Formula (I) as claimed in Claim 1, wherein a 2-methylnaphthylamide of the Formula

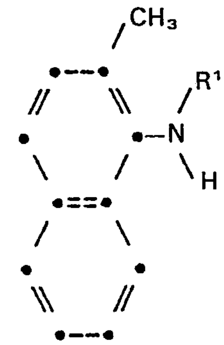 (II)

where $R^1$ has the meanings given in Claim 1, is reacted

a) with an acid halide of the Formula

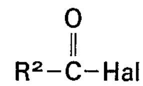 (III)

where $R^2$ has the meanings given in Claim 1, and Hal is chlorine or bromine, or

b) with an acid anhydride of the Formula

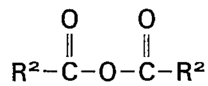 (IV)

where $R^2$ had the meanings given in Claim 1, in the presence or absence of a solvent or diluent, with or without the addition of an inorganic or organic base, and with or without the addition of an accelerator, at from −10 to +100° C.

7. An N-substituted 2-methylnaphthylamide of the Formula (I) as claimed in Claim 1, in which $R^2$ is isothiazol-3-, -4- or -5-yl, 3-methylisothiazol-4-yl, 3-methylisothiazol-5-yl, thiazol-2-, -4- or -5-yl, 4-methylthiazol-5-yl, 1,2,3-thiadiazol-4-yl, 1,2,4-thiadiazol-3-yl, 1,2,4-thiadiazol-5-yl, 1,2,5-thiadiazol-3-yl or 1,3,4-thiadiazol-2-yl.

8. A fungicide containing an N-substituted 2-methylnaphthylamide of the Formula (I) as claimed in Claim 1, in which $R^2$ is isothiazol-3-, -4- or -5-yl, 3-methylisothiazol-4-yl, 3-methylisothiazol-5-yl, thiazol-2-, -4- or -5-yl, 4-methylthiazol-5-yl, 1,2,3-thiadiazol-4-yl, 1,2,4-thiadiazol-3-yl, 1,2,4-thiadiazol-5-yl, 1,2,5-thiadiazol-3-yl or 1,3,4-thiadiazol-2-yl.

**Claims** for the Contracting State: AT

1. A fungicide containing an N-substituted 2-methylnaphthylamide of the Formula

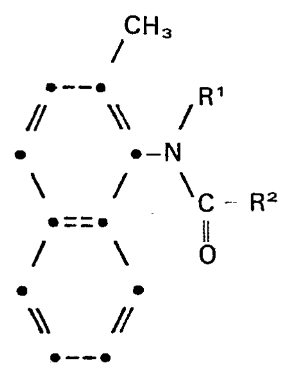 (I)

where $R^1$ is $-CH(CH_3)-COOCH_3$, and $R^2$ is a 5-membered heterocyclic ring which contains a sulfur atom and not more than two nitrogen atoms and is unsubstituted or substituted by methyl or ethyl, with the exception of 1,2,3-thiadiazol-5-yl.

2. A fungicide containing a solid or liquid carrier and an N-substituted 2-methylnaphthylamide of the Formula (I) as defined in Claim 1.

3. A process for producing a fungicide, wherein a solid or liquid carrier is mixed with an N-substituted 2-methylnaphthylamide of the Formula (I) as defined in Claim 1.

4. A process for combating fungi, wherein the fungi or the objects to be protected against fungus attack are treated with an N-substituted 2-methylnaphthylamide of Formula (I) as defined in Claim 1.

5. A process for preparing an N-substituted 2-methylnaphthylamide of the Formula (I) as defined in Claim 1, wherein a 2-methylnaphthylamide of the Formula

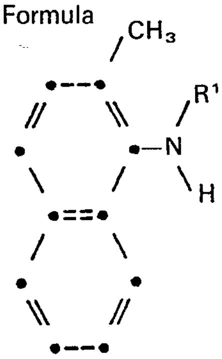

(II)

where $R^1$ has the meanings given in Claim 1, is reacted

a) with an acid halide of the Formula

O
||
$R^2-C-Hal$ (III)

where $R^2$ has the meanings given in Claim 1, and Hal is chlorine or bromine, or

b) with an acide anhydride of the Formula

O O
|| ||
$R^2-C-O-C-R^2$ (IV)

where $R^2$ has the meanings given in Claim 1, in the presence or absence of a solvent or diluent, with or without the addition of an inorganic or organic base, and with or without the addition of an accelerator, at from −10 to +100° C.

6. A fungicide containing N-substituted 2-methylnaphthylamide of the Formula (I) as defined in Claim 1, in which $R^2$ is isothiazol-3-, -4- or -5-yl, 3-methylisothiazol-4-yl, 3-methylisothiazol-5-yl, thiazol-2-, -4- or -5-yl, 4-methylthiazol-5-yl, 1,2,3-thiadiazol-4-yl, 1,2,4-thiadiazol-3-yl, 1,2,4-thiadiazol-5-yl, 1,2,5-thiadiazol-3-yl or 1,3,4-thiadiazol-2-yl.

**Revendications** pour les Etats contractants BE, CH, DE, FR, GB, IT, LI, NL, SE

1. 2-Méthyl-naphtyl-amide N-substitué de la formule

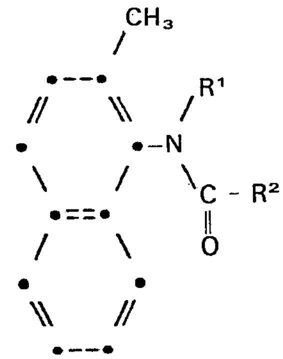

(I)

dans laquelle

$R^1$ est un groupe $-CH(CH_3)-COOCH_3$, et

$R^2$ désigne un noyau hétérocyclique pentagonal, contenant un atome de soufre et un ou deux atomes d'azote, éventuellement méthyl- ou éthyl-substitué, à l'exception du groupe 1,2,3-thiadiazol-5-yle.

2. Fongicide, contenant un 2-méthyl-naphtyl-amide N-substitué de la formule (I) selon la revendication 1.

3. Fongicide, contenant un 2-méthyl-naphthyl-amide N-substitué de la formule (I) selon la revendication 1 et un véhicule solide ou liquide.

4. Procédé de préparation d'un fongicide, caractérisé en ce que l'on mélange un 2-méthyl-naphtyl-amide N-substitué de la formule (I) selon la revendication 1 et un véhicule solide ou liquide.

5. Procédé de lutte contre des champignons, caractérisé en ce que l'on traite les champignons ou les objets à protéger des champignons avec un 2-méthyl-naphtyl-amide N-substitué de la formule (I) selon la revendication 1.

6. Procédé de préparation d'un 2-méthyl-naphtyl-amide N-substitué de la formule (I) selon la revendication 1, caractérisé en ce que l'on fait réagir un 2-méthyl-naphtyl-amide de la formule

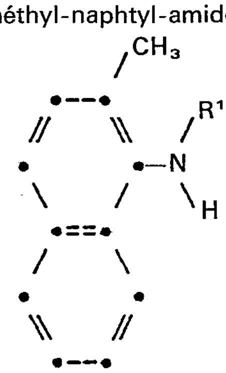

(II)

dans laquelle $R^1$ possède la signification définie dans la revendication 1 soit

a) avec un halogénure d'acide de la formule

O
||
$R^2-C-Hal$ (III)

dans laquelle $R^2$ possède les significations définies dans la revendication 1 et Hal désigne un atome de chlore ou de brome, soit

b) avec un anhydride d'acide de la formule

O O
|| ||
$R^2-C-O-C-R^2$ (IV)

dans laquelle $R^2$ possède les significations définies dans la revendication 1, à des températures comprises entre −10 et +100° C, éventuellement dans un solvant et en présence d'une base inorganique ou organique et/ou d'un accélérateur de la réaction.

7. 2-Méthyl-naphtyl-amides N-substitués de la formule (I) selon la revendication 1, caractérisé en ce que $R^2$ désigne un groupe 3-, 4- ou 5-isothiazolyle, 3-méthyl-4-isothiazolyle, 3-méthyl-5-isothiazolyle, 2-, 4- ou 5-thiazolyle, 4-méthyl-5-thiazolyle, 1,2,3-thiadiazol-4-yle, 1,2,4-thiadiazol-3-yle, 1,2,4-thiadiazol-5-yle, 1,2,5-thiadiazol-3-yle ou 1,3,4-thiadiazol-2-yle.

8. Fongicide, contenant un 2-méthyl-naphtyl-amide N-substitué de la formule (I) selon la revendication 1, caractérisé en ce que $R^2$ désigne un groupe 3-, 4- ou 5-isothiazolyle, 3-méthyl-4-isothiazolyle, 3-méthyl-5-isothiazolyle, 2-, 4- ou 5-thiazolyle, 4-méthyl-5-thiazolyle, 1,2,3-thiadiazol-4-yle, 1,2,4-thiadiazol-3-yle, 1,2,4-thiadiazol-5-yle, 1,2,5-thiadiazol-3-yle ou 1,3,4-thiadiazol-2-yle.

**Revendications** pour l'Etat contractant: AT

1. Fongicide, contenant un 2-méthyl-naphtyl-amide N-substitué de la formule

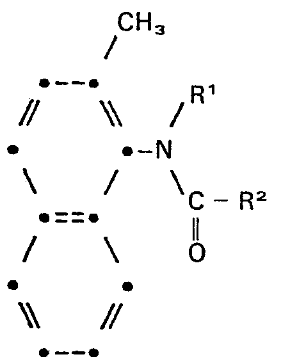

(I)

dans laquelle

$R^1$ est un groupe $-CH(CH_3)-COOCH_3$, et

$R^2$ désigne un noyau hétérocyclique pentagonal, contenant un atome de soufre et un ou deux atomes d'azote, éventuellement méthyl- ou éthyl-substitué, à l'exception du groupe 1,2,3-thiadiazol-5-yle.

2. Fongicide, contenant un 2-méthyl-naphtyl-amide N-substitué de la formule (I) selon la revendication 1 et un véhicule solide ou liquide.

3. Procédé de préparation d'un fongicide, caractérisé en ce que l'on mélange un 2-méthyl-naphtyl-amide N-substitué de la formule (I) selon la revendication 1 avec un véhicule solide ou liquide.

4. Procédé de lutte contre des champignons, caractérisé en ce que l'on traite les champignons ou les objets à protéger des champignons avec un 2-méthyl-naphtyl-amide N-substitué de la formule (I) selon la revendication 1.

5. Procédé de préparation d'un 2-méthyl-naphtyl-amide N-substitué de la formule (I) selon la revendication 1, caractérisé en ce que l'on fait réagir un 2-méthyl-naphtyl-amide de la formule

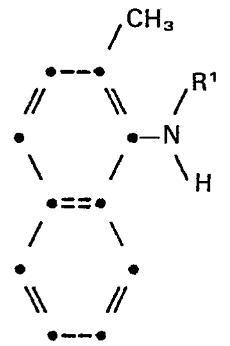

(II)

dans laquelle $R^1$ possède la signification définie dans la revendication 1, soit

a) avec un halogénure d'acide de la formule

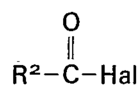

(III)

dans laquelle $R^2$ possède les significations définies dans la revendication 1 et Hal désigne un atome de chlore ou de brome, soit

b) avec un anhydride d'acide de la formule

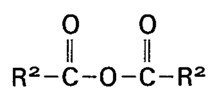

(IV)

dans laquelle $R^2$ possède les significations définies dans la revendication 1, à des températures comprises entre −10 et +100° C, éventuellement dans un solvant et en présence d'une base inorganique ou organique et/ou d'un accélérateur de la réaction.

6. Fongicide, contenant un 2-méthyl-naphtyl-amide N-substitué de la formule (I), caractérisé en ce que $R^2$ désigne un groupe 3-, 4- ou 5-isothiazolyle, 3-méthyl-4-isothiazolyle, 3-méthyl-5-isothiazolyle, 2-, 4- ou 5-thiazolyle, 4-méthyl-5-thiazolyle, 1,2,3-thiadiazol-4-yle, 1,2,4-thiadiazol-3-yle, 1,2,4-thiadiazol-5-yle, 1,2,5-thiadiazol-3-yle ou 1,3,4-thiadiazol-2-yle.